# EUROPEAN PATENT SPECIFICATION

(11) **EP 3 517 618 B1**
(45) Date of publication and mention of the grant of the patent: **06.09.2023**
(21) Application number: 16916823.4
(22) Date of filing: 26.09.2016
(51) Int. Cl.: C12P 7/06, B01D 8/00, B01D 53/26, B01D 53/44, C12M 1/00, C12P 7/54

(54) **METHOD AND APPARATUS FOR PRODUCING VALUABLE SUBSTANCE**
VERFAHREN UND VORRICHTUNG ZUR HERSTELLUNG EINER WERTVOLLEN SUBSTANZ
PROCÉDÉ ET APPAREIL DE PRODUCTION D'UNE SUBSTANCE DE VALEUR

(43) Date of publication of application: 31.07.2019
(73) Proprietor: Sekisui Chemical Co., Ltd., Osaka-shi, Osaka 530-8565 (JP)
(72) Inventor: OKADA, Kazumi, Tsukuba-shi Ibaraki 300-4292 (JP); FUJIMORI, Yoji, Tsukuba-shi Ibaraki 300-4292 (JP)
(74) Representative: Ter Meer Steinmeister & Partner
(86) International application number: PCT/JP2016/078215
(87) International publication number: WO 2018/055756

(56) References cited:
- EP-A1- 3 045 538
- WO-A1-2009/058028
- WO-A1-2013/081779
- WO-A1-2015/037710
- WO-A1-2015/081331
- JP-A- H07 108 102
- JP-A- 2008 163 256
- JP-A- 2011 144 330
- JP-A- 2013 221 062
- JP-A- 2016 187 332
- US-A1- 2012 068 120
- JEFFREY HUFTON ET AL: "Advanced hydrogen and COcapture technology for sour syngas", ENERGY PROCEDIA, vol. 4, 2011, pages 1082-1089, XP028213018, ISSN: 1876-6102, DOI: 10.1016/J.EGYPRO.2011.01.158 [retrieved on 2011-04-01]

## Description

### FIELD OF THE INVENTION

The present invention relates to a method and an apparatus for producing valuable substances such as ethanol from a raw material gas such as a synthetic gas, and particularly relates to a method and an apparatus suitable for producing valuable substances from a synthetic gas derived from wastes. The invention is defined in the appended claims.

### BACKGROUND OF THE INVENTION

For example, in Patent Document 1, a synthetic gas is introduced to a culture tank and valuable substances such as ethanol are produced by microbial fermentation. Patent Document 1 mentions that the synthetic gas contains small amounts of oxygen, particulate matters, tar, H₂S, BTEX (benzene, toluene, ethyl benzene, xylene) or the like; these substances may be harmful to microorganism; and therefore, are removed in a pretreatment section.

In Patent Document 2, moisture in a gas generated by thermally decomposing wastes is reduced in a gas dehumidifying device. When chemicals adhere to the gas dehumidifying device, causing a pressure loss, a high-temperature gas is sent to the gas dehumidifying device to eliminate the chemicals by evaporation or sublimation.

### PRIOR ART DOCUMENTS

### PATENT DOCUMENTS

Patent Document 1: Japanese Patent Application Publication No. 2014-050406 ([0102])
Patent Document 2: Publication of Japanese Patent No. 4551774

### SUMMARY OF THE INVENTION

### PROBLEM TO BE SOLVED BY THE INVENTION

A study conducted by the inventor et al. found that a synthetic gas (raw material gas) derived from wastes or the like may contain phase transition impurity substances that can change phases between a gas phase and a solid phase, including sublimation substances such as naphthalene, 1-naphtol, 2-naphtol or the like. This kind of phase transition impurity substances may be in the gas phase when generated in a waste disposal facility (raw material gas generator), but may be solid-phased depending on temperature conditions or the like in a supply passage to a culture tank (valuable substance producing reactor) and adhere to a filter or the like. The synthetic gas derived from wastes usually contains a large amount of moisture content, and if an endeavor is made to reduce the moisture content by cooling, the phase transition impurity substances will be more prone to be solid-phased. If the solid-phased phase transition impurity substances are caught by a filter together with solid or liquid impurity substances such as soot and tar, the filter will be easy to be clogged, requiring more frequent filter exchange. This makes maintenance work more complicated, with increased operating cost. On the other hand, when the solid-phased phase transition impurity substances are vaporized again by hot air or the like as in the Patent Document 2, a gas containing the gas-phased phase transition impurity substances introduced to a valuable substance producing reactor may have adverse effects on a valuable substance producing reaction.

In view of the knowledge and consideration given above, it is an object of the present invention to avoid or reduce various problems or adverse effects by phase transition impurity substances when valuable substances are produced from a synthetic gas containing the phase transition impurity substances that can change phases in a supply passage from a generator of a raw material gas such as a synthetic gas to a valuable substance producing reactor.

### MEANS FOR SOLVING THE PROBLEMS

To solve the problems mentioned above, the present invention provides a method for producing a valuable substance from a raw material gas, including: a phase transition impurity substance removing step, wherein the raw material gas from a raw material gas generator is passed through a phase transition impurity substance remover to remove a phase transition impurity substance from the raw material gas, the phase transition impurity substance changing phases between a gas phase and a solid phase; a solid/liquid catching step, wherein the raw material gas after the phase transition impurity substance removing step is passed through a solid/liquid catcher to catch a solid or liquid impurity substance in the raw material gas; and a reacting step, wherein the raw material gas after the solid/liquid catching step is introduced to a valuable substance producing reactor, where a reaction occurs to produce the valuable substance.

According to the method for producing the valuable substances, the phase transition impurity substances such as naphthalene in the raw material gas can be removed before introducing the raw material gas to the solid/liquid catcher, and further to the valuable substance producing reactor. Thereby, various problems or adverse effects by phase transition impurity substances can be avoided or reduced, such as the solid/liquid catcher becoming less likely to be clogged and the phase transition impurity substances being prevented from entering into the valuable substance producing reactor.

To "remove" impurity substances means removing at least a portion of the impurity substances from the raw material gas, and is not limited to completely removing the impurity substances from the raw material gas.

The phase transition impurity substance removing step includes: a first cooling step, wherein the raw material gas is passed through a first gas cooler, the raw material gas being cooled in the first gas cooler; and a phase transition impurity substance catching step, wherein the cooled raw material gas is passed through a phase transition impurity substance catcher that catches a solidified substance of the phase transition impurity substance.

The phase transition impurity substances such as naphthalene can be solid-phased by cooling. After that, the solidified substance of the phase transition impurity substances can be caught by the phase transition impurity substance catcher and can be removed.

After the phase transition impurity substance catching step and before the solid/liquid catching step, a second cooling step is performed, wherein the raw material gas is passed through a second gas cooler, where a temperature of the raw material gas is made lower than a temperature of the raw material gas in the first cooling step.

By performing the second cooling step, moisture contained in the raw material gas can be sufficiently condensed and removed before the raw material gas is introduced to the solid/liquid catcher. Thereby, the solid/liquid catcher can become less likely to be clogged by the moisture. By removing the phase transition impurity substances beforehand, the solid/liquid catcher can become further less likely to be clogged.

In the reacting step, gas-utilizing microorganisms are cultured in a liquid culture medium, the raw material gas is supplied to the culture medium, and the valuable substance is produced by fermentation of the gas-utilizing microorganisms.

By supplying the raw material gas having the phase transition impurity substances sufficiently removed in the phase transition impurity substance removing step, the gas-utilizing microorganism can be cultured in a stable manner.

The present invention provides an apparatus for producing a valuable substance from a raw material gas produced by a raw material gas generator, the apparatus including: a phase transition impurity substance remover that removes a phase transition impurity substance from the raw material gas, the phase transition impurity substance changing phases between a gas phase and a solid phase; a solid/liquid catcher that catches a solid or liquid impurity substance in the raw material gas; and a valuable substance producing reactor, where a reaction occurs to produce the valuable substance from the raw material gas, wherein the phase transition impurity substance remover, the solid/liquid catcher and the valuable substance producing reactor are arranged along a supply passage of the raw material gas in this order from an upstream side.

According to the apparatus for producing the valuable substance, the phase transition impurity substances are removed from the raw material gas by the phase transition impurity substance remover. Subsequently, the solid or liquid impurity substances in the raw material gas are caught by the solid/liquid catcher. After that, the reaction occurs to produce the valuable substances from the raw material gas in the valuable substance producing reactor. By this arrangement, various problems or adverse effects by phase transition impurity substances can be avoided or reduced, such as the solid/liquid catcher becoming less likely to be clogged and the phase transition impurity substances being prevented from entering into the valuable substance producing reactor.

The phase transition impurity substance remover includes: a first cooler that cools the raw material gas; and a phase transition impurity substance catcher that catches a solidified substance of the phase transition impurity substance, wherein the first cooler and the phase transition impurity substance catcher are arranged along the supply passage in this order from the upstream side.

The phase transition impurity substances such as naphthalene can be solid-phased by cooling the raw material gas by the first cooler. After that, the solidified substance of the phase transition impurity substances can be caught by the phase transition impurity substance catcher.

Preferably, a filter of the phase transition impurity substance catcher is coarser than a filter of the solid/liquid catcher.

By making the filter of the phase transition impurity substance catcher coarse, the filter can become less likely to be clogged by the solidified substance of the phase transition impurity substances or by the condensed water in some cases.

By making the filter of the solid/liquid catcher fine, the solid/liquid impurity substances such as soot and tar can be surely caught and removed. By removing a majority of the phase transition impurity substances by the phase transition impurity substance catcher beforehand, the filter of the solid/liquid catcher can become less likely to be clogged. The solidified substance of the phase transition impurity substances that penetrated the phase transition impurity substance catcher can be surely caught by the solid/liquid catcher and removed.

The second cooler is provided between the phase transition impurity substance catcher and the solid/liquid catcher along the supply passage, the second cooler making a temperature of the raw material gas lower than a temperature of the raw material gas in the first cooler.

Thereby, the moisture contained in the raw material gas can be sufficiently condensed and removed before the raw material gas is introduced to the solid/liquid catcher. Thereby, the solid/liquid catcher can become less likely to be clogged by the moisture.

The first cooler includes a first cooling passage, the raw material gas being cooled while being passed through the first cooling passage, the second cooler includes a second cooling passage, the raw material gas being cooled while being passed through the second cooling passage, and a conductance of the first cooling passage is higher than a conductance of the second cooling passage.

By making the conductance of the first cooling passage high, the first cooling passage can become less likely to be clogged by the solidified substance of the phase transition impurity substances. On the other hand, by making the conductance of the second cooling passage low and further by making a diameter of a cooling tube constituting the second cooling passage small, a cooling efficiency of the second cooling passage to cool the raw material gas can be enhanced and the moisture contained in the raw material gas can be surely condensed and removed. Furthermore, the solid/liquid catcher can surely become less likely to be clogged by the moisture.

The valuable substance producing reactor includes a culture tank, gas-utilizing microorganisms being cultured in a liquid culture medium in the culture tank, the raw material gas is supplied to the culture medium, and the valuable substance is produced by fermentation of the gas-utilizing microorganisms.

By supplying the raw material gas having the phase transition impurity substances sufficiently removed by the phase transition impurity substance remover to the culture medium, the gas-utilizing microorganisms can be cultured in a stable manner.

### ADVANTAGEOUS EFFECTS OF THE INVENTION

According to the present invention, various problems or adverse effects by phase transition impurity substances in a raw material gas can be avoided or reduced when valuable substances such as ethanol are produced from the raw material gas.

### BRIEF DESCRIPTION OF THE DRAWINGS

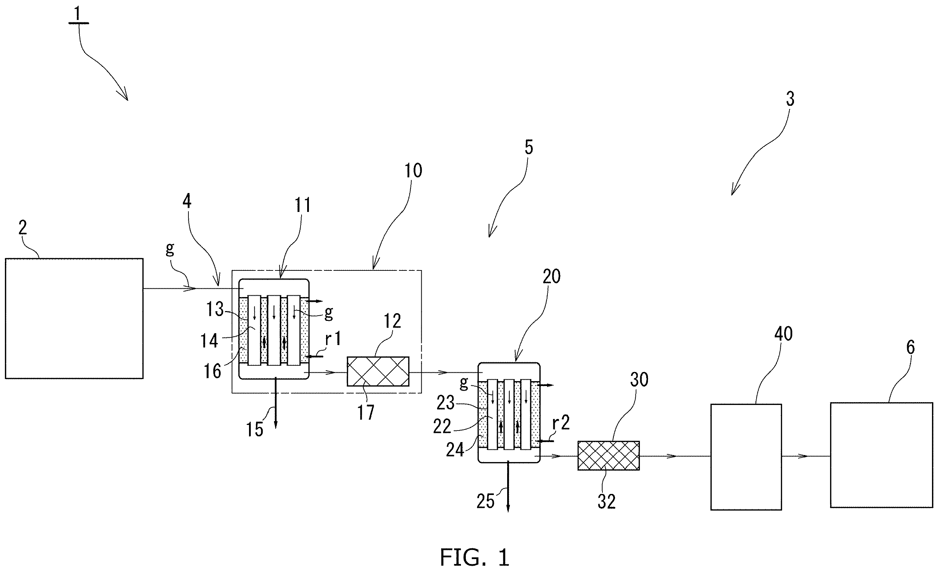

FIG. 1 is a schematic drawing of a valuable substance producing system according to one embodiment of the present invention.

### MODE FOR CARRYING OUT THE INVENTION

One embodiment of the present invention is described hereinafter.

As shown in FIG. 1, a valuable substance producing system 1 includes a synthetic gas generator 2 (raw material gas generator) and a valuable substance producing apparatus 3. A synthetic gas g (raw material gas) is generated in the synthetic gas generator 2. Valuable substances are produced from the synthetic gas g in the valuable substance producing apparatus 3. The valuable substance as a desired product may be ethanol (C₂H₅OH), for example.

The synthetic gas generator 2 in this embodiment is a waste disposal facility. Wastes may include municipal wastes, tires, biomass, wooden chips and plastic wastes. A melting furnace is provided in the waste disposal facility 2. The wastes are burnt by a highly concentrated oxygen gas and decomposed to a low-molecular level in the melting furnace. Eventually, the synthetic gas g is generated. A temperature of the synthetic gas g at the time of generation or at the time of supply from the synthetic gas generator 2 is higher than an ordinary temperature, which may be around 30 degrees to several hundred degrees C, for example.

The synthetic gas g derived from wastes includes CO, H₂ and CO₂ as main components. Moreover, the synthetic gas g contains a lot of water (H₂O). Usually, this kind of synthetic gas g is generally saturated with water. Moreover, the synthetic gas g contains solid or liquid impurity substances such as soot or tar and phase transition impurity substances in addition to gaseous impurity substances such as H₂S, O₂ and BTEX as impurity substances harmful to or unsuitable for production of valuable substances. The phase transition impurity substances mentioned here means impurity substances (excluding water) that are mostly in a gas phase at the time of supply from the synthetic gas generator 2, but can change phases in the course of transportation to a valuable substance producing reactor 6 to be described later to be partly or entirely in a solid phase. Examples of such phase transition impurity substances may include sublimation substances such as naphthalene, 1-naphtol and 2-naphthol.

The valuable substance producing apparatus 3 includes a purifier 5 and a valuable substance producing reactor 6. A supply passage 4 extends from the synthetic gas generator 2 to the valuable substance producing reactor 6. The purifier 5 is interposed in the supply passage 4.

The purifier 5 includes a phase transition impurity substance remover 10, a second cooler 20, a solid/liquid catcher 30 and a gaseous impurity substance remover 40. The phase transition impurity substance remover 10 includes a first cooler 11 and a phase transition impurity substance catcher 12. The first cooler 11, the phase transition impurity substance catcher 12, the second cooler 20, the solid/liquid catcher 30, the gaseous impurity substance remover 40 and the valuable substance producing reactor 6 are disposed along the supply passage 4 arranged from an upstream side (the synthetic gas generator 2 side, left side in FIG. 1) in this order. That is, the phase transition impurity substance remover 10, the solid/liquid catcher 30 and the valuable substance producing reactor 6 are arranged in this order.

The first cooler 11 is composed of a heat exchanger (chiller), for example. Preferably, the first cooler 11 is composed of a multitubular heat exchanger including a plurality of cooling tubes 13. Preferably, the cooling tubes 13 are straight tubes in view of the ease with which it is washed. The cooling tubes 13 are arranged parallel to each other with tube axes thereof oriented vertically.

An inside of the cooling tube 13 serves as a first cooling passage 14. The first cooling passage 14 is interposed in the supply passage 4. The synthetic gas g is passed through the first cooling passage 14.

A drain passage 15 extends from a bottom portion of the first cooler 11.

A space external to the cooling tubes 13 or a space between the cooling tubes 13, 13 in the first cooler 11 serves as a refrigerant passage 16. A refrigerant r1 such as ethylene glycol is flown in the refrigerant passage 16. A flow of the synthetic gas g in the first cooling passage 14 and a flow of the refrigerant r1 in the refrigerant passage 16 are countercurrent to each other. Alternatively, they may be parallel currents.

A cooling target temperature of the first cooler 11 is set in a range in which the phase transition (sublimation and solidification of naphthalene, for example) can well happen. The cooling target temperature of the first cooler 11 is in a vicinity of an ordinary temperature, specifically around 10 to 30 degrees C, and more preferably, around 20 degrees C.

The phase transition impurity substance catcher 12 is disposed downstream (right side in FIG. 1) of the first cooler 11 along the supply passage 4. The phase transition impurity substance catcher 12 includes a phase transition impurity substance catching filter 17. The filter 17 may be made of metal, resin, fiber, ceramic or a mixture thereof. The filter 17 may have a multi-layer mesh structure.

A second cooler 20 is disposed downstream (right side in FIG. 1) of the phase transition impurity substance catcher 12 along the supply passage 4. The second cooler 20 is composed of a heat exchanger (chiller), for example. Preferably, the second cooler 20 is composed of a multitubular heat exchanger including a plurality of cooling tubes 23. Preferably, the cooling tubes 23 are straight tubes in view of the ease with which it is washed. The cooling tubes 23 are arranged parallel to each other with tube axes thereof oriented vertically.

An inside of the cooling tube 23 serves as a second cooling passage 22. The second cooling passage 22 is interposed in the supply passage 4. The synthetic gas g is passed through the second cooling passage 22.

A drain passage 25 extends from a bottom portion of the second cooler 20.

A space external to the cooling tubes 23 or a space between the cooling tubes 23, 23 in the second cooler 20 serves as a refrigerant passage 24. A refrigerant r2 such as ethylene glycol is flown in the refrigerant passage 24. A flow of the synthetic gas g in the second cooling passage 22 and a flow of the refrigerant r2 in the refrigerant passage 24 are countercurrent to each other. Alternatively, they may be parallel currents.

A cooling target temperature of the second cooler 20 is set in a range in which the moisture in the synthetic gas g can be sufficiently condensed (liquefied) and yet not frozen. The cooling target temperature of the second cooler 20 is not lower than 0 degrees C and not higher than the ordinary temperature, specifically around 0 to 10 degrees C, and more preferably, around 2 degrees C.

When the first cooler 11 and the second cooler 20 are compared, the cooling target temperature of the first cooler 11 is higher than that of the second cooler 20. A diameter of the cooling tube 13 of the first cooler 11 is larger than a diameter of the cooling tube 23 of the second cooler 20. Accordingly, a conductance of the first cooling passage 14 is higher than a conductance of the second cooling passage 22.

The solid/liquid catcher 30 is disposed downstream of the second cooler 20 along the supply passage 4. The solid/liquid catcher 30 includes a solid/liquid catching filter 32. The filter 32 may be made of metal, resin, fiber, ceramic or a mixture thereof. The filter 32 may have a multi-layer mesh structure.

When the phase transition impurity substance catcher 12 and the solid/liquid catcher 30 are compared, the filter 17 of the phase transition impurity substance catcher 12 is coarser than the filter 32 of the solid/liquid catcher 30. Accordingly, it is easier for particles to pass through the filter 17 than the filter 32. When both of the filter 17 and the filter 32 have multi-layer mesh structures, the number of mesh layers of the filter 17 is preferably one third to one twentieth of the number of mesh layers of the filter 32, and more preferably not greater than one tenth of the number of mesh layers of the filter 32.

The gaseous impurity substance remover 40 is disposed downstream of the solid/liquid catcher 30 along the supply passage 4. The gaseous impurity substance remover 40 includes a pressure-swing adsorption (PSA) device and a metallic catalyst. Adsorbent for the PSA device may include zeolite, silica gel and activated carbon. The metallic catalyst may include Cu and Pd.

A downstream end of the supply passage 4 is connected to the valuable substance producing reactor 6. Although not shown in detail in the drawing, the valuable substance producing reactor 6 includes a culture tank. Anaerobic gas-utilizing microorganisms are cultured in a liquid culture medium in the culture tank. As the gas-utilizing microorganisms, anaerobic bacteria disclosed in the Patent Document 1 mentioned above, the International Patent Application Publication No. WO2011/087380 and the US Patent Application Publication No. US2013/0065282, etc. may be used.

Although not shown in the drawing, a refiner including a distiller is provided in a subsequent stage of the valuable substance producing reactor 6.

A method for producing ethanol (valuable substance) by the valuable substance producing system 1 is described below.

### <Synthetic Gas Generating Step>

The synthetic gas g is generated by burning the wastes in the synthetic gas generator 2. The synthetic gas g is introduced to the supply passage 4. As mentioned above, the temperature of the synthetic gas g when being introduced is higher than the ordinary temperature.

### <Phase Transition Impurity Substance Removing Step>

The synthetic gas g is passed through the phase transition impurity substance remover 10 to remove the naphthalene, 1-naphtol and other phase transition impurity substances (hereinafter referred to as "naphthalene or the like" as appropriate) in the synthetic gas g.

### <First Cooling Step>

Specifically, the synthetic gas g is cooled in the first cooler 11. That is, the synthetic gas g is passed through the first cooling passage 14 and the refrigerant r1 is passed through the refrigerant passage 16, and heat is exchanged therebetween. Thereby, the synthetic gas g is cooled to around the ordinary temperature, for example. By being cooled, the naphthalene or the like in the synthetic gas g is sublimated (solid phased) from gas to solid.

By making the diameter of the cooling tube 13 relatively large and making the conductance of the first cooling passage 14 relatively high, the first cooling passage 14 can become less likely to be clogged by the solidified substance of naphthalene or the like.

A portion of the moisture in the synthetic gas g is condensed by the cooling. The condensed water is eliminated through the drain passage 15.

### <Phase Transition Impurity Substance Catching Step>

The cooled synthetic gas g is sent to the phase transition impurity substance catcher 12 from the first cooler 11 and passed through the filter 17. Thereby, solidified substances such as the naphthalene or the like in the synthetic gas g can be caught by the filter 17, and the naphthalene or the like can be removed from the synthetic gas g. By making the filter 17 relatively coarse, the filter 17 can become less likely to be clogged by the solidified substances such as the naphthalene or the like or the condensed water, thereby, frequency of exchanging the filter 17 can be reduced.

### <Second Cooling Step>

Subsequently, the synthetic gas g is introduced to the second cooler 20 to be cooled further. That is, the synthetic gas g is passed through the second cooling passage 22 and the refrigerant r2 is passed through the refrigerant passage 24, and heat is exchanged therebetween. Thereby, the synthetic gas g is cooled further than in the first cooling step to preferably around zero degree C. By making the diameter of the cooling tube 13 small, cooling efficiency of the synthetic gas g can be enhanced. Thereby, the moisture in the synthetic gas g can be sufficiently condensed, and a moisture content of the synthetic gas g can be made generally zero.

By performing the second cooling step by the second cooler 20 after removing the naphthalene or the like by the phase transition impurity substance remover 10 beforehand, the second cooling passage 22 can become less likely to be clogged.

A portion of the water condensed by the second cooler 20 is eliminated through the drain passage 25.

### < Solid/Liquid Catching Step>

Subsequently, the synthetic gas g is sent to the solid/liquid catcher 30 and passed through the filter 32. Solid/liquid impurity substances such as the soot and tar in the synthetic gas g can be caught by the filter 32 and removed.

By performing the solid/liquid catching step by the solid/liquid catcher 30 after removing the moisture in the synthetic gas g by the second cooler 20 beforehand, the filter 32 can become less likely to be clogged.

Furthermore, by performing the solid/liquid catching step by the solid/liquid catcher 30 after removing the naphthalene or the like by the phase transition impurity substance remover 10 beforehand, the filter 32 can become further surely less likely to be clogged.

The solidified substance of the naphthalene or the like that passed through the phase transition impurity substance remover 10 can be caught by the solid/liquid catcher 30.

### <Other Impurity Substance Removing Step>

Subsequently, the synthetic gas g is introduced to the gaseous impurity substance remover 40. The gaseous impurity substances such as H₂S, O₂ and BTEX are removed by the gaseous impurity substance remover 40.

As a result, the synthetic gas g can be made clean.

### <Reacting Step>

Subsequently, the synthetic gas g is introduced to culture liquid in the valuable substance producing reactor 6. In the valuable substance producing reactor 6, the gas-utilizing microorganisms in the culture liquid intake CO and H₂ or the like in the synthetic gas g and produce ethanol or the like by fermentation. That is, a reaction occurs to produce ethanol (valuable substance) from the synthetic gas g as a raw material.

By removing the gaseous impurity substances such as O₂, BTEX and H₂S and solid/liquid impurity substances such as the soot and tar in the synthetic gas g beforehand, the gas-utilizing microorganisms can be cultured in a stable manner.

Furthermore, by removing the phase transition impurity substances such as the naphthalene or the like beforehand, the gas-utilizing microorganisms can be cultured in a more stable manner.

### <Refining Step>

A portion of the culture liquid is introduced to the distiller (not shown) and distilled there. Thereby, ethanol can be extracted.

As mentioned above, in the valuable substance producing apparatus 3, by removing the phase transition impurity substances such as the naphthalene or the like from the synthetic gas g, troubles such as clogging and adverse effects to the reaction to produce valuable substances by the phase transition impurity substances can be avoided or reduced.

By making the filters 17, 32 finer in stages while cooling the synthetic gas g in stages by the two-stage (multiple-stage) coolers 11, 20, various kinds of impurity substances can be efficiently removed.

When the first cooling passage 14 starts to clog, it is recommended to wash the passage by back washing or the like. Since the cooling tubes 13 are straight tubes, the washing can be easily performed. The same applies to the second cooling passages 22.

When the filter 17 starts to clog, the filter should be exchanged. The same applies to the filter 32. As mentioned above, in the valuable substance producing apparatus 3, the filters 17, 32 can become less likely to be clogged, and exchange frequency can be reduced. Therefore, maintenance can be simplified, and operating cost can be reduced.

The present invention is not limited to the embodiments described above. Various modifications can be made without departing from the scope of the invention.

For example, the phase transition impurity substance remover 10 may include an oil scrubber. The phase transition impurity substances such as naphthalene may be removed by the oil scrubber.

The phase transition impurity substances are not limited to sublimation substances such as naphthalene, 1-naphtol, 2-naphtol that change phases from a gas phase to a solid phase, not through a liquid phase. The phase transition impurity substances may be those that change phases from a gas phase to a solid phase through a liquid phase.

The valuable substance as a desired product is not limited to ethanol, but may be acetic acid or methanol or the like.

The valuable substance producing reactor 6 may produce valuable substances such as ethanol by bringing the synthetic gas g into contact with a metallic catalyst.

The synthetic gas generator 2 is not limited to the waste disposal facility, but may be a steel plant, a coal power plant or the like. The raw material gas may be a by-product gas (converter gas, blast furnace gas or the like) from a steel plant.

### INDUSTRIAL APPLICABILITY

The present invention may be applied to an ethanol producing system, wherein ethanol is synthesized from a syngas generated in an incineration treatment of industrial wastes, for example.

### EXPLANATION OF REFERENCE NUMERALS

- 1: valuable substance producing system
- 2: synthetic gas generator (raw material gas generator)
- 3: valuable substance producing apparatus
- 4: supply passage
- 5: purifier
- 6: valuable substance producing reactor
- 10: phase transition impurity substance remover
- 11: first cooler
- 12: phase transition impurity substance catcher
- 13: cooling tube
- 14: first cooling passage
- 15: drain passage
- 16: refrigerant passage
- 17: phase transition impurity substance catching filter
- 20: second cooler
- 23: cooling tube
- 22: second cooling passage
- 24: refrigerant passage
- 25: drain passage
- 30: solid/liquid catcher
- 32: solid/liquid catching filter
- 40: gaseous impurity substance remover
- g: synthetic gas (raw material gas)
- r1: refrigerant
- r2: refrigerant

## Claims

1. A method for producing a valuable substance from a raw material gas, **CHARACTERIZED IN THAT** the method comprises:
a phase transition impurity substance removing step, wherein the raw material gas from a raw material gas generator (2) is passed through a phase transition impurity substance remover (10) to remove a phase transition impurity substance from the raw material gas, the phase transition impurity substance changing phases between a gas phase and a solid phase;
a solid/liquid catching step, wherein the raw material gas after the phase transition impurity substance removing step is passed through a solid/liquid catcher (30) to catch a solid or liquid impurity substance in the raw material gas; and
a reacting step, wherein the raw material gas after the solid/liquid catching step is introduced to a valuable substance producing reactor (6), where a reaction occurs to produce the valuable substance; wherein the phase transition impurity substance removing step comprises:
a first cooling step, wherein the raw material gas is passed through a first gas cooler (11), the raw material gas being cooled in the first gas cooler (11); and
a phase transition impurity substance catching step, wherein the cooled raw material gas is passed through a phase transition impurity substance catcher (12) that catches a solidified substance of the phase transition impurity substance; wherein after the phase transition impurity substance catching step and before the solid/liquid catching step, a second cooling step is performed, wherein the raw material gas is passed through a second gas cooler (20), where a temperature of the raw material gas is made lower than a temperature of the raw material gas in the first cooling step; and
wherein a cooling target temperature of the first gas cooler (11) is around 10 to 30 degrees C, and a cooling target temperature of the second gas cooler (20) is around 0 to 10 degrees C.

2. The method for producing the valuable substance according to claim 1, wherein in the reacting step, gas-utilizing microorganisms are cultured in a liquid culture medium, the raw material gas is supplied to the culture medium, and the valuable substance is produced by fermentation of the gas-utilizing microorganisms.

3. An apparatus (3) for producing a valuable substance from a raw material gas produced by a raw material gas generator (2), **CHARACTERIZED IN THAT** the apparatus (3) comprises:
a phase transition impurity substance remover (10) that removes a phase transition impurity substance from the raw material gas, the phase transition impurity substance changing phases between a gas phase and a solid phase;
a solid/liquid catcher (30) that catches a solid or liquid impurity substance in the raw material gas; and
a valuable substance producing reactor (6), where a reaction occurs to produce the valuable substance from the raw material gas,
wherein the phase transition impurity substance remover (10), the solid/liquid catcher (30) and the valuable substance producing reactor (6) are arranged along a supply passage (4) of the raw material gas in this order from an upstream side; wherein the phase transition impurity substance remover (10) comprises:
a first cooler (11) that cools the raw material gas; and
a phase transition impurity substance catcher (12) that catches a solidified substance of the phase transition impurity substance,
wherein the first cooler (11) and the phase transition impurity substance catcher (12) are arranged along the supply passage (4) in this order from the upstream side;
wherein a second cooler (20) is provided between the phase transition impurity substance catcher (12) and the solid/liquid catcher (30) along the supply passage (4), the second cooler (20) making a temperature of the raw material gas lower than a temperature of the raw material gas in the first cooler (11); and wherein a cooling target temperature of the first gas cooler (11) is around 10 to 30 degrees C, and a cooling target temperature of the second gas cooler (20) is around 0 to 10 degrees C.

4. The apparatus (3) for producing the valuable substance according to claim 3, wherein a filter (17) of the phase transition impurity substance catcher (12) is coarser than a filter (32) of the solid/liquid catcher (30).

5. The apparatus (3) for producing the valuable substance according to claim 3 or 4, wherein
the first cooler (11) includes a first cooling passage (14), the raw material gas being cooled while being passed through the first cooling passage (14),
the second cooler (20) includes a second cooling passage (22), the raw material gas being cooled while being passed through the second cooling passage (22), and
a conductance of the first cooling passage (14) is higher than a conductance of the second cooling passage (22).

6. The apparatus (3) for producing the valuable substance according to any one of claims 3 to 5, wherein
the valuable substance producing reactor (6) includes a culture tank (6), gas-utilizing microorganisms being cultured in a liquid culture medium in the culture tank (6),
the raw material gas is supplied to the culture medium, and
the valuable substance is produced by fermentation of the gas-utilizing microorganisms.

## Patentansprüche

1. Verfahren zur Herstellung einer wertvollen Substanz aus einem Rohmaterialgas, **dadurch gekennzeichnet, dass** das Verfahren umfasst:
einen Phasenübergangsverunreinigungssubstanz-Entfernungsschritt, bei dem das Rohmaterialgas von einem Rohmaterialgasgenerator (2) durch einen Phasenübergangsverunreinigungssubstanz-Entferner (10) geleitet wird, um eine Phasenübergangsverunreinigungssubstanz aus dem Rohmaterialgas zu entfernen, wobei die Phasenübergangsverunreinigungssubstanz die Phasen zwischen einer Gasphase und einer festen Phase wechselt;
einen Feststoff/Flüssigkeits-Auffangschritt, wobei das Rohmaterialgas nach dem Phasenübergangsverunreinigungssubstanz-Entfernungsschritt durch einen Feststoff/Flüssigkeits-Fänger (30) geleitet wird, um eine feste oder flüssige Verunreinigungssubstanz in dem Rohmaterialgas aufzufangen; und
einen Reaktionsschritt, wobei das Rohmaterialgas nach dem Feststoff/Flüssigkeits-Auffangschritt in einen Wertstofferzeugungsreaktor (6) eingeleitet wird, in dem eine Reaktion stattfindet, um die wertvolle Substanz zu erzeugen; wobei der Phasenübergangsverunreinigungssubstanz-Entfernungsschritt umfasst:
einen ersten Kühlschritt, wobei das Rohmaterialgas durch einen ersten Gaskühler (11) geleitet wird, wobei das Rohmaterialgas in dem ersten Gaskühler (11) gekühlt wird; und
einen Phasenübergangsverunreinigungssubstanz-Auffangschritt, wobei das gekühlte Rohmaterialgas durch einen Phasenübergangsverunreinigungssubstanz-Fänger (12) geleitet wird, der eine verfestigte Substanz der Phasenübergangsverunreinigungssubstanz auffängt; wobei nach dem Phasenübergangsverunreinigungssubstanz-Auffangschritt und vor dem Feststoff/Flüssigkeits-Auffangschritt ein zweiter Kühlschritt durchgeführt wird, wobei das Rohmaterialgas durch einen zweiten Gaskühler (20) geleitet wird, wobei eine Temperatur des Rohmaterialgases niedriger gemacht wird als eine Temperatur des Rohmaterialgases im ersten Kühlschritt; und
wobei eine Kühlzieltemperatur des ersten Gaskühlers (11) etwa 10 bis 30 Grad C beträgt und eine Kühlzieltemperatur des zweiten Gaskühlers (20) etwa 0 bis 10 Grad C beträgt.

2. Verfahren zur Herstellung der wertvollen Substanz nach Anspruch 1, wobei im Reaktionsschritt gasverwertende Mikroorganismen in einem flüssigen Kulturmedium kultiviert werden, das Rohmaterialgas dem Kulturmedium zugeführt wird und die wertvolle Substanz durch Fermentation der gasverwertenden Mikroorganismen hergestellt wird.

3. Vorrichtung (3) zur Herstellung einer wertvollen Substanz aus einem Rohmaterialgas, das von einem Rohmaterialgasgenerator (2) erzeugt wird, **dadurch gekennzeichnet, dass** die Vorrichtung (3) Folgendes umfasst:
einen Phasenübergangsverunreinigungssubstanz-Entferner (10), der eine Phasenübergangsverunreinigungssubstanz aus dem Rohmaterialgas entfernt, wobei die Phasenübergangsverunreinigungssubstanz die Phasen zwischen einer Gasphase und einer festen Phase wechselt;
einen Feststoff/Flüssigkeits-Fänger (30), der eine feste oder flüssige Verunreinigungssubstanz in dem Rohmaterialgas auffängt; und
einen Wertstofferzeugungsreaktor (6), in dem eine Reaktion stattfindet, um die wertvolle Substanz aus dem Rohmaterialgas zu erzeugen,
wobei der Phasenübergangsverunreinigungssubstanz-Entferner (10), der Feststoff/Flüssigkeits-Fänger (30) und der Wertstofferzeugungsreaktor (6) entlang eines Zufuhrkanals (4) des Rohmaterialgases in dieser Reihenfolge von einer stromaufwärts gelegenen Seite aus angeordnet sind; wobei der Phasenübergangsverunreinigungssubstanz-Entferner (10) umfasst:
einen ersten Kühler (11), der das Rohmaterialgas kühlt; und
einen Phasenübergangsverunreinigungssubstanz-Fänger (12), der eine verfestigte Substanz der Phasenübergangsverunreinigungssubstanz auffängt,
wobei der erste Kühler (11) und der Phasenübergangsverunreinigungssubstanz-Fänger (12) entlang des Zufuhrkanals (4) in dieser Reihenfolge von der stromaufwärts gelegenen Seite aus angeordnet sind;
wobei ein zweiter Kühler (20) zwischen dem Phasenübergangsverunreinigungssubstanz-Fänger (12) und dem Feststoff/Flüssigkeits-Fänger (30) entlang des Zufuhrkanals (4) vorgesehen ist, wobei der zweite Kühler (20) eine Temperatur des Rohmaterialgases niedriger macht als eine Temperatur des Rohmaterialgases in dem ersten Kühler (11); und
wobei eine Kühlungszieltemperatur des ersten Gaskühlers (11) etwa 10 bis 30 Grad C beträgt und eine Kühlungszieltemperatur des zweiten Gaskühlers (20) etwa 0 bis 10 Grad C beträgt.

4. Vorrichtung (3) zur Herstellung der wertvollen Substanz nach Anspruch 3, wobei ein Filter (17) des Phasenübergangsverunreinigungssubstanz-Fängers (12) gröber ist als ein Filter (32) des Feststoff/Flüssigkeits-Fängers (30).

5. Vorrichtung (3) zur Herstellung der wertvollen Substanz nach Anspruch 3 oder 4, wobei der erste Kühler (11) einen ersten Kühlkanal (14) aufweist, wobei das Rohmaterialgas gekühlt wird, während es durch den ersten Kühlkanal (14) geleitet wird,
der zweite Kühler (20) einen zweiten Kühlkanal (22) aufweist, wobei das Rohmaterialgas gekühlt wird, während es durch den zweiten Kühlkanal (22) geleitet wird, und
ein Leitwert des ersten Kühlkanals (14) höher ist als ein Leitwert des zweiten Kühlkanals (22).

6. Vorrichtung (3) zur Herstellung der wertvollen Substanz nach einem der Ansprüche 3 bis 5, wobei
der Wertstofferzeugungsreaktor (6) einen Kulturtank (6) enthält, wobei in dem Kulturtank (6) gasverwertende Mikroorganismen in einem flüssigen Kulturmedium kultiviert werden,
das Rohmaterialgas dem Kulturmedium zugeführt wird, und
die wertvolle Substanz durch Fermentation der gasverwertenden Mikroorganismen erzeugt wird.

## Revendications

1. Procédé pour produire une substance valorisable à partir d'une matière première gazeuse, **caractérisé en ce que** le procédé comporte :
une étape de retrait de substance d'impureté de transition de phase, dans laquelle la matière première gazeuse provenant d'un générateur de matière première gazeuse (2) passe à travers un dispositif de retrait de substance d'impureté de transition de phase (10) pour retirer une substance d'impureté de transition de phase de la matière première gazeuse, la substance d'impureté de transition de phase changeant des phases entre une phase gazeuse et une phase solide ;
une étape de capture de solide/liquide, dans laquelle la matière première gazeuse après l'étape de retrait de substance d'impureté de transition de phase passe à travers un dispositif de capture de solide/liquide (30) pour capturer une substance d'impureté solide ou liquide dans la matière première gazeuse ; et
une étape de réaction, dans laquelle la matière première gazeuse après l'étape de capture de solide/liquide est introduite dans un réacteur de production de substance valorisable (6), où une réaction a lieu pour produire la substance valorisable ; dans lequel l'étape de retrait de substance d'impureté de transition de phase comporte :
une première étape de refroidissement, dans laquelle la matière première gazeuse passe à travers un premier refroidisseur de gaz (11), la matière première gazeuse étant refroidie dans le premier refroidisseur de gaz (11) ; et
une étape de capture de substance d'impureté de transition de phase, dans laquelle la matière première gazeuse refroidie passe à travers un dispositif de capture de substance d'impureté de transition de phase (12) qui capture une substance solidifiée de la substance impureté de transition de phase ; dans lequel après l'étape de capture de substance d'impureté de transition de phase et avant l'étape de capture de solide/liquide, une seconde étape de refroidissement est réalisée, dans laquelle la matière première gazeuse passe à travers un second refroidisseur de gaz (20), où une température du matière première gazeuse est abaissée par rapport à une température de la matière première gazeuse à la première étape de refroidissement ; et
dans lequel une température cible de refroidissement du premier refroidisseur de gaz (11) est d'environ 10 à 30 degrés C, et une température cible de refroidissement du second refroidisseur de gaz (20) est d'environ 0 à 10 degrés C.

2. Procédé pour produire la substance valorisable selon la revendication 1, dans lequel à l'étape de réaction, des micro-organismes consommateurs de gaz sont mis en culture dans un milieu de culture liquide, la matière première gazeuse est fournie au milieu de culture, et la substance valorisable est produite par fermentation des micro-organismes consommateurs de gaz.

3. Appareil (3) pour produire une substance valorisable à partir d'une matière première gazeuse produite par un générateur de matière première gazeuse (2), **caractérisé en ce que** l'appareil (3) comporte :
un dispositif de retrait de substance d'impureté de transition de phase (10) qui retire une substance d'impureté de transition de phase de la matière première gazeuse, la substance d'impureté de transition de phase changeant des phases entre une phase gazeuse et une phase solide ;
un dispositif de capture de solide/liquide (30) qui capture une substance d'impureté solide ou liquide dans la matière première gazeuse ; et
un réacteur de production de substance valorisable (6), où une réaction a lieu pour produire la substance valorisable à partir de la matière première gazeuse,
dans lequel le dispositif de retrait de substance d'impureté de transition de phase (10), le dispositif de capture de solide/liquide (30) et le réacteur de production de substance valorisable (6) sont agencés le long d'un passage d'alimentation (4) de la matière première gazeuse dans cet ordre depuis un côté amont ; dans lequel le dispositif de retrait de substance d'impureté de transition de phase (10) comporte :
un premier refroidisseur (11) qui refroidit la matière première gazeuse ; et
un dispositif de capture de substance d'impureté de transition de phase (12) qui capture une substance solidifiée de la substance d'impureté de transition de phase,
dans lequel le premier refroidisseur (11) et le dispositif de capture de substance d'impureté de transition de phase (12) sont agencés le long du passage d'alimentation (4) dans cet ordre depuis le côté amont ;
dans lequel un second refroidisseur (20) est agencé entre le dispositif de capture de substance d'impureté de transition de phase (12) et le dispositif de capture de solide/liquide (30) le long du passage d'alimentation (4), le second refroidisseur (20) abaissant une température de la matière première gazeuse par rapport à une température de la matière première gazeuse dans le premier refroidisseur (11) ; et dans lequel une température cible de refroidissement du premier refroidisseur de gaz (11) est d'environ 10 à 30 degrés C, et une température cible de refroidissement du second refroidisseur de gaz (20) est d'environ 0 à 10 degrés C.

4. Appareil (3) pour produire la substance valorisable selon la revendication 3, dans lequel un filtre (17) du dispositif de capture de substance d'impureté de transition de phase (12) est plus grossier qu'un filtre (32) du dispositif de capture de solide/liquide (30).

5. Appareil (3) pour produire la substance valorisable selon la revendication 3 ou 4, dans lequel
le premier refroidisseur (11) inclut un premier passage de refroidissement (14), la matière première gazeuse étant refroidie tout en passant à travers le premier passage de refroidissement (14),
le second refroidisseur (20) inclut un second passage de refroidissement (22), la matière première gazeuse étant refroidie tout en passant à travers le second passage de refroidissement (22), et
une conductance du premier passage de refroidissement (14) est supérieure à une conductance du second passage de refroidissement (22).

6. Appareil (3) pour produire la substance valorisable selon l'une quelconque des revendications 3 à 5, dans lequel
le réacteur de production de substance valorisable (6) inclut un réservoir de culture (6), des micro-organismes consommateurs de gaz étant mis en culture dans un milieu de culture liquide dans le réservoir de culture (6),
la matière première gazeuse est fournie au milieu de culture, et
la substance valorisable est produite par fermentation des micro-organismes consommateurs de gaz.
